# EUROPEAN PATENT SPECIFICATION

(11) **EP 0 402 652 B1**
(45) Date of publication and mention of the grant of the patent: **08.12.1993**
(21) Application number: 90109368.2
(22) Date of filing: 17.05.1990
(51) Int. Cl.: C07C 21/18, C07C 17/20, C07C 17/00

(54) **Gas phase catalytic process for production of vinylidene fluoride**
Katalytisches Gasphasenverfahren zur Herstellung von Vinylfluorid
Procédé catalytique en phase gazeuse pour la préparation de fluorure de vinylidène

(30) Priority: 13.06.1989 US 365621
(43) Date of publication of application: 19.12.1990
(73) Proprietor: ATOCHEM NORTH AMERICA, INC., Philadelphia, Pennsylvania 19102 (US)
(72) Inventor: Elsheikh, Maher Yousef, Wayne, Pennsylvania 19087 (US); Bolmer, Michael Sheppard, Collegeville, Pennsylvania 19426 (US)
(74) Representative: Kraus, Walter, Dr.

(56) References cited:
- DE-B- 1 288 085
- DE-B- 1 288 593
- US-A- 4 827 055

## Description

The invention relates to the production of vinyli- dene fluoride. More particularly, the invention relates to a gas phase catalytic process for the hydrofluorination of 1,1,1-trichloroethane and/or vinylidene chloride to produce vinylidene fluoride.

US-A-4 827 055 discloses a process for the preparation of vinylidene fluoride, wherein vinylidene chloride is reacted with HF in the gas phase at a temperature of from 400° to 700°C in the presence of oxygen and an anhydrous AlF₃ containing catalyst. 1,1,1-Trifluoroethane is a waste by-product. It may be recovered and dehydrofluorinated to form vinylidene fluoride. See Japanese Published Patent Application 54/130507. Alternatively, the 1,1,1-trifluoroethane is incinerated.

While the dehydrofluorination of 1,1,1-trifluoroethane to generate vinylidene fluoride is feasible, other uses of 1,1,1-trifluoroethane are sought.

It has not been heretofore recognized that hydrofluorocarbons may be utilized as diluents and catalyst activators to improve the performance of gas phase fluorination catalysts, in particular catalysts utilized in the hydrofluorination of 1,1,1-trichloroethane or vinylidene chloride.

A process for producing vinylidene fluoride is provided. A vapor phase reaction mixture is formed comprising hydrogen fluoride and at least one halohydrocarbon of the formula CHₙCXₘ wherein n and m are 2 or 3 and each X is independently selected from the group of fluorine, chlorine, bromine and iodine. 1,1,1-Trifluoroethane is added to the vapor phase mixture as a diluent, and hydrogen fluoride and the halohydrocarbon react in the presence of a heterogeneous fluorination catalyst. Vinylidene fluoride is obtained as a reaction product. Preferably, the 1,1,1-trifluoroethane added to the reaction mixture comprises a hydrofluorination reaction by-product which is separated from vinylidene fluoride and recycled back to the reaction mixture.

According to one embodiment of the invention, a continuous process for the production of vinylidene fluoride is provided. At least one halohydrocarbon as defined above is continuously fed to a reaction zone. Hydrogen fluoride and the halohydrocarbon are continuously reacted in the vapor phase in the reaction zone in the presence of a heterogeneous fluorination catalyst. A product mixture comprising vinylidene fluoride and 1,1,1-trifluoroethane is continuously withdrawn from the reaction zone, and at least a portion of the 1,1,1-trifluoroethane is recycled back to the reaction zone.

We have found that 1,1,1-trifluoroethane, heretofore regarded as a waste by-product in the gas phase catalytic hydrofluorination of CHₙCXₘ halohydrocarbons, may be utilized as a diluent and catalyst activator to improve the selectively for the desired product, vinylidene fluoride, in the hydrofluorination reaction. Thus, it is believed that the addition of 1,1,1-trifluoroethane back to the reaction mixture improves the performance of the heterogeneous hydrofluorination catalyst. According to the present invention, the selectively for vinylidene fluoride may be increased by 10-15%, depending on the operating conditions.

Generally, hydrogen fluoride and the CHₙCXₘ halohydrocarbon are fed to the reaction zone at a molar ratio of from 0:1 to 10:1, preferably from 1:1 to 4:1. The feed of hydrogen fluoride may be reduced to zero in some circumstances by virtue of the in situ generation of hydrogen fluoride by the dehydrofluorination of 1,1,1-trifluoroethane, which proceeds simultaneously with the hydrofluorination of the CHₙCXₘ halohydrocarbon. The reactants are combined in a reaction zone in the gaseous state. The reaction zone may comprise any of the known reactor vessels suitable for gas phase hydrofluorination. Such devices are well known to those skilled in the art.

The process may be operated at any temperature favoring the conversion of the halohydrocarbon to vinylidene fluoride. Generally, the process is operated at a temperature of from 400°C to 650°C, preferably from 550°C to 625°C. The pressure is not critical, provided it is not so great that the reactants would be liquified at the reaction temperature.

The residence time of the reaction mixture in the reaction zone at the reaction temperature may be selected in accordance with the desired extent of conversion of halohydrocarbon to vinylidene fluoride. Typically, the reactants are in contact in the reaction zone for a period of time from 1 second to 20 seconds, preferably from 5 to 20 seconds.

The feed may comprise any saturated or olefinic halohydrocarbon wherein all of the hydrogen atoms are substituted onto one carbon, and all of the halogen atoms are substituted onto the other carbon. Preferably, the halohydrocarbon comprises vinylidene chloride and/or 1,1,1-trichloroethane. Additional halohydrocarbon compounds readily catalytically hydrofluorinated to vinylidene fluoride include, for example, 1-chloro-1-fluoroethylene, 1,1-dichloro-1-fluoroethane, 1-chloro-1,1-difluoroethane. 1-Chloro-1-fluoroethylene, 1,1-dichloro-1-fluoroethane and 1-chloro-1,1-difluoroethane may be generated as intermediates in the hydrofluorination of 1,1,1-trichloroethane or vinylidene chloride.

The reaction is generally carried out in the presence of a suitable heterogeneous catalyst. The catalyst advantageously comprises a metal fluoride, for example, AlF₃, CrF₃, FeF₃, SbS₅, NiF₂, BF₃, SnF₄, and combinations thereof. Other suitable catalysts for use in the process include metal oxides or metal chlorides which are converted to the corresponding metal fluorides upon activation with hydrogen fluoride. Thus, included in the catalysts which may be used in the herein process are compounds which may be converted to metal fluorides at the herein described reaction conditions. Such catalysts include, by way of example, FeCl₃, Fe₂O₃, CoCl₂, CoO, CrCl₃, Cr₂O₃. The catalyst may be used directly, or may be carried on an appropriate catalyst support, such as γ-aluminum oxide. Such supported catalysts may be employed, for example, in the form of pellets or granules.

While the ratio of hydrogen fluoride to CHₙCXₘ halohydrocarbon advantageously fed to the reaction zone is from 1:1 to 10:1, the amount of hydrogen fluoride delivered to the reaction zone may, in some circumstances, be satisfactorily reduced to zero, as sufficient hydrogen fluoride may be generated to sustain the hydrofluorination of the halohydrocarbon feed by the simultaneous dehydrofluorination of 1,1,1-trifluoroethane. The amount of hydrogen fluoride required to sustain the production of vinylidene fluoride at satisfactory levels should therefore necessarily take into account the amount of hydrogen fluoride generated in situ as a dehydrofluorination product. For example, two moles of 1,1,1-trifluoroethane will generate sufficient hydrogen fluoride to convert one mole of vinylidene chloride to vinylidene fluoride. In some circumstances, sufficient 1,1,1-trifluoroethane may be supplied to the reaction zone from an independent source such that the hydrogen fluoride requirement for sustaining the hydrofluorination reaction may be satisfied by the hydrogen fluoride formed in situ by the dehydrofluorination of 1,1,1-trifluoroethane. In such circumstances a separate hydrogen fluoride feed in unnecessary. On the other hand, if the 1,1,1-trifluoroethane introduced into the reaction zone is not sourced from outside the reaction system, but rather merely comprises a 1,1,1-trifluoroethane recycle feed, hydrogen fluoride from an independent source must be supplied to sustain the hydrofluorination reaction for extended periods.

An oxidizing agent may optionally be introduced into the reaction zone to insure that the catalyst remains free of carbonaceous deposits or "coke". Thus, an oxidizing gas may be introduced into the reactor to accelerate the combustion of any carbonaceous deposits which may accumulate on the catalyst surface as a result of the burning of organic reactants. Such oxidizing gases may include, for example, O₂ and/or CO₂. The oxidizing gas is advantageously combined with the feed halohydrocarbon as it is fed to the reaction zone. Where O₂ is used as the oxidizing agent, the ratio of O₂ to the halohydrocarbon may vary from about 0 to about 20 mol%, preferably from 5 to 10 mold. Where CO₂ is used as the oxidizing gas, the ratio of CO₂ to halohydrocarbon may vary from 0 to 200 mol%, preferably from 50 to 100 mol%.

The process of the invention may be carried out as a batch, semi-continuous, or continuous process. Preferably, the process is conducted in a continuous fashion comprising continuously feeding hydrogen fluoride and the halohydrocarbon to the reaction zone to form a vapor phase reaction mixture thereof. Hydrogen fluoride and the halohydrocarbon are continuously reacted in the presence of the heterogeneous catalyst. A product mixture comprising vinylidene fluoride and 1,1,1-trifluoroethane is continuously withdrawn from the reaction zone. After separation of vinylidene fluoride, at least a portion of the 1,1,1-trifluoroethane is recycled back to the reaction zone.

Any amount of 1,1,1-trifluoroethane may be recycled back to the reaction mixture. Preferably, substantially all the 1,1,1-trifluoroethane by-product of the gas phase hydrofluorination reaction is recycled back to the reaction zone, to maximize efficiency. When 1,1,1-trifluoroethane from an independent source is utilized, the amount added to the reaction mixture may vary considerably. Even the smallest amount will provide a benefit. Amounts of 1,1,1-trifluoroethane in excess of 70 mol%, based on the combined amount of hydrogen fluoride and the feed halohydrocarbon, are generally not preferred since the other components of the reaction mixture may begin to become too dilute.

In a typical continuous operation, hydrogen fluoride and the feed halohydrocarbon are passed through a tubular reactor loaded with a hydrogen fluoride-activated catalyst. The reaction products, being predominantly a mixture of vinylidene fluoride and 1,1,1-trifluoroethane, are continuously removed from the reactor as gas phase products, and passed to a suitable scrubbing tower for removal of HCl and HF by the action of a countercurrent alkaline stream. The alkaline stream may comprise, for example, 16% potassium hydroxide solution. Other agueous hydroxides such as sodium hydroxide or calcium hydroxide may be advantageously utilized. The scrubbed product is then passed to a drying tower which is packed with a suitable drying agent, e.g. anhydrous calcium sulfate.

Vinylidene fluoride is readily separated from the other components of the reactor effluent gas by conventional techniques such as distillation, and the like, preferably after removal of hydrogen fluoride and hydrogen chloride. The effluent gas, comprising predominantly vinylidene fluoride and 1,1,1-trifluoroethane, the feed halohydrocarbon, minor amounts of intermediates such as chlorofluoroethylene, 1,1-dichloro-1-fluoroethane, 1-chloro-1,1-difluoroethane, and also the oxidizing gas, is subjected to a separation process which preferentially separates vinylidene fluoride. Separation is most advantageously achieved by distilling vinylidene fluoride, which has a boiling point of -86°C, from the product mixture.

Separation of vinylidene fluoride may be accomplished in a conventional distillation column which is operated, for example, at a temperature and pressure at the column top of about -4°C and 285 PSIG, respectively, and a bottom temperature and pressure of about 38°C and 280 PSIG, respectively. Under these conditions, vinylidene fluoride is readily separated from 1,1,1-trifluoroethane, which has a boiling point of -48°C, and from the various feed halohydrocarbons and reaction intermediates, which boil at temperatures substantially higher than the -86°C boiling point of vinylidene fluoride. At least a portion of the distillation bottom product, which comprises 1,1,1-trifluoroethane, the feed halohydrocarbon and hydrofluorination intermediates, is advantageously recycled to the reaction mixture. While the oxidizing gas is taken overhead with the vinylidene fluoride, the two may be thereafter separated by simple distillation.

The materials of construction of the reactor for use in the present process should possess the necessary structural and physical characteristics to withstand the reaction conditions.

The present invention is illustrated in more detail by reference to the following examples.

### Comparative Example 1

A 7.5 wt.% FeF₃/AlF₃ catalyst was prepared as follows and utilized for the conversion of vinylidene chloride to vinylidene fluoride.

Alumina (200 g, 1.96 mol) was added portionwise to a magnetically-stirred solution of 52% by weight of aqueous HF (500 ml, 15 mol). Addition of alumina was controlled to keep the temperature of the reaction mixture between 40° and 45°C. The addition took seven hours. The resulting milky suspension was left at room temperature overnight. Upon standing, a fine powder (presumably AlF₃. 9H₂O) precipitated. A solution of 37.5 g FeCl₃ (0.23 mol) in 20 ml H₂O was added gradually to the mechanically-stirred agueous AlF₃, mixture. The solution turned a light violet color. The stirring was continued for four hours and the mixture was left to settle at room temperature overnight. The product precipitate was filtered and washed with acetone several times until the filtrate was acid free. The solid obtained was dried in air at room temperature and was heated at 100°C for 2 hours, 150°C for 2 hours and finally at 175°C for 16 hours. The resulting FeF₃/AlF₃ (7.5 wt.% FeF₃/AlF₃) catalyst was ground using a mortar and pestle, and was sieved. The 0.25 to 0.149 mm particles were collected and used to fill a nickel, fixed bed tubular reactor 2.54 cm outside diameter x 30.48 cm. The bed was heated using a single zone furnace. The catalyst was gradually heated to 650°C at the rate of 100°C per hour, using air fed to the reactor at the rate of 20 cm³/min. The catalyst was maintained at this temperature and air flow for two and one half days. The temperature of the reactor was then lowered to 550°C, and hydrogen fluoride at the rate of 0.03 g/min. and N₂ at the rate of 20 ml/min. were passed over the catalyst for 18 hours. The temperature was raised to 650°C and a mixture of 15 mol% vinylidene chloride, 54 mol% hydrogen fluoride, 1 mol% O₂ and 30 mol% N₂ were fed to the reactor through valves located at the reactor top. The product was withdrawn, and after scrubbing HCl and excess HF with a 16% KOH solution, the scrubbed product was dried with anhydrous CaSO₄. Analysis of the product by gas chromatography indicated a 34 mol% conversion of vinylidene chloride. Selectivity for vinylidene fluoride was 15 mol%.

### Example 1

The procedure of Comparative Example 1 was repeated, except that the N₂ was replaced with a like amount of 1,1,1-trifluoroethane. Conversion of vinylidene chloride increased to 100 mol%. Selectivity for vinylidene fluoride was calculated as net vinylidene fluoride selectivity, by subtracting from the product the amount of 1,1,1-trifluoroethane which was added in the reactor feed. The net vinylidene fluoride selectivity was 23 mol%. The balance of the product distribution was as follows: 1,1,1-trifluoroethane, 73 mol%; 1,1-difluoro-1-chloroethane, 3 mol%; and 1-chloro-1-fluoroethylene, 1 mol%.

It is apparent from a study of Comparative Example 1 and Example 1 that fluorination of vinylidene chloride increased from 32 mol% to 91 mol%, upon substitution of 1,1,1-trifluoroethane for the diluent (100% fluorination being defined as complete conversion of vinylidene chloride to 1,1,1-trifluoroethane).

The mass balance in the reactor was computed by dividing the measured flow rate of material from the reactor by the measured flow rate of material into the reactor. The mass balance for Comparative Example 1, using N₂ is the diluent, was 87%. Substituting 1,1,1-trifluoroethane as the diluent in Example 1 resulted in a mass balance of 95%. This means that the amount of product lost as coke on the catalyst was reduced from 13% to 5%, when 1,1,1-trifluoroethane was substituted for N₂ as the reaction diluent.

### Comparative Example 2

A 6.4 wt.% NiCl₂/AlF₃ catalyst was prepared as follows and utilized in the conversion of vinylidene chloride to vinylidene fluoride.

Nicl₂ (29.94 grams in 150 ml of water was added to precipitated AlF₃ from aqueous hydrogen fluoride (as described in Comparative Example 1). After drying and sieving the catalyst, 20 grams of the 0.25 - 0.149 mm size particles were loaded into the same reactor as used in Comparative Example 1. The catalyst was air activated for two days at 650°C, using air fed to the reactor at the rate of 20 cm³/min. The catalyst was then activated by hydrogen fluoride fed at the rate of 0.03 g/min. for 18 hours at 553°C. A mixture of 1,1,1-trichloroethane, (10 mol%), HF (77 mol%), O₂ (2 mol%), N₂ (8 mol%) and BF₃ (3 mol%) was fed from the top of the reactor at 550°C. After removing HF and HCl, and drying the product as previously described in Comparative Example 1, analysis of the product gas by gas chromatography indicated 96 mol% conversion of 1,1,1-trichloroethane, with the following product distribution: 7 mol% vinylidene fluoride; 34 mol% vinylidene chloride; 17 mol % 1-chloro-1-fluoroethylene; 34 mol% 1,1,1-trifluoroethane ; 4 mol% 1-chloro-1,1-difluoroethane; and 4 mol% 1-fluoro-1,1-dichloroethane.

### Example 2

Comparative Example 2 is repeated except that the hydrogen fluoride feed was reduced, and 1,1,1-trifluoroethane was added to the reaction mixture as a diluent. The composition of the feed was as follows: 12 mol% 1,1,1-trichloroethane; 49 mol% HF; 3 mol% O₂; 10 mol% N₂; 4 mol% BF₃ ; and 22 mol% 1,1,1-trifluoroethane. The conversion of 1,1,1-trifluoroethane achieved was 99 mol%. The net selectivity for vinylidene fluoride was 23 mol%, with the remaining product distribution comprising 20 mol% vinylidene chloride, 4 mol% 1-chloro-1-fluoroethylene, 50 mol% 1,1,1-trifluoroethane, 1 mol% 1-chloro-1,1-difluoroethane, and 2 mol% 1,1-dichloro-1-fluoroethane.

From a consideration of Example 2 and Comparative Example 2, it may be appreciated that upon addition of 1,1,1-trifluoroethane as a reaction diluent, total fluorination of 1,1,1-trichloroethane increased from 47 mol% to 67 mol%, despite the fact that the hydrogen fluoride/1,1,1-trichloroethane molar ratio was decreased from 7.7:1 to 4.1:1. The calculated loss of product as catalyst coke decreased from 90% to 49%.

### Comparative Example 3

A catalyst comprising 3.35 wt.% SbF₅ and 6.1 wt.% NiF₂/AlF₃ was prepared as follows and utilized in the conversion of vinylidene chloride to vinylidene fluoride.

A solution of NiCl₂ (29.94 g, 0.23 mol) in 50 ml of water was added to AlF₃ precipitated from Al₂O₃ (200 g) and hydrogen fluoride (500 ml, 50%) according to Comparative Example 1. The NiCl₂ solution was added to the AlF₃ with continuous stirring. SbCl₅ (22.4 g, 0.075 mol) was added portionwise over a three hour period. The resulting mixture was left standing overnight to precipitate. The mixture was filtered and acetone-washed. The filter cake was collected, air dried, then heated in an oven at 100°C for 2 hours, at 150°C for 2 hours and finally at 175°C for 18 hours. The resulting catalyst was ground, sieved, and 34.7 grams of the 60-80 mesh particles were collected and loaded into the reactor as described previously in Comparative Example 1. A mixture of 9.5 mol% 1,1,1-trichloroethane, 39 mol% hydrogen fluoride, 0.5 mol% O₂ and 51 mol% N₂ was fed into the reactor. Conversion of 1,1,1-trichloroethane was 100%. The product distribution was as follows: vinylidene fluoride, 2 mol%; vinylidene chloride, 79 mol%; 1-chloro-1-fluoroethylene, 14 mol%, 1,1,1-trifluoroethane, 2 mol%; other products, 3 mol%.

### Example 3

Comparative Example 3 was repeated, except that the reactor feed comprised the following mixture: 13 mol% 1,1,1-trichloroethane; 49 mol% HF; 3 mol% O₂; 11 mol% N₂; and 24 mol% 1,1,1-trifluoroethane. Conversion of 1,1,1-trichloroethane was 94 mol%. The net selectivity for vinylidene fluoride was 48% mol. The remaining product distribution was as follows: 1,1,1-trifluoroethane, 51 mol%; and 1-chloro-1,1-difluoroethane, 1 mol%.

From a comparison of Comparative Example 3 with Example 3, it is observed that the total fluorination of 1,1,1-trichloroethane increased from 8 mold to 79 mol% upon the addition of 1,1,1-trifluoroethane as a diluent.

## Claims

1. A process for producing vinylidene fluoride comprising:
forming a vapor phase reaction mixture comprising hydrogen fluoride and at least one halohydrocarbon of the formula CHₙCXₘ wherein n and m are 2 or 3 and each X is independently selected from the group of fluorine, chlorine, bromine and iodine;
adding 1,1,1-trifluoroethane and/or at least a portion of 1,1,1-trifluoroethane produced as a reaction product in said process back to the vapor phase reaction mixture as a diluent;
reacting the hydrogen fluoride and halohydrocarbon in a reaction zone in the presence of a heterogeneous catalyst; and
obtaining vinylidene fluoride as a reaction product.

2. The process according to claim 1 wherein the halohydrocarbon is selected from the group of vinylidene chloride, 1,1,1-trichloroethane and mixtures thereof.

3. The process according to claim 1 or 2 wherein the reaction temperature in the reaction zone is from 400°C to 650°C.

4. The process according to claim 3 wherein the reaction temperature in the reaction zone is from 550°C to 625°C.

5. The process according to any one of claims 1 to 4 wherein the halohydrocarbon and hydrogen fluoride are in contact in the reaction zone for a period of time from 1 second to 20 seconds.

6. The process according to claim 5 wherein the halohydrocarbon and hydrogen fluoride are in contact in the reaction zone for a period of time from 5 seconds to 20 seconds.

7. The process according to any one of claims 1 to 6 wherein the vapor phase reaction mixture is formed by feeding hydrogen fluoride and the halohydrocarbon to the reaction zone in a molar feed ratio of from 0:1 to 10:1.

8. The process according to claim 7 wherein the molar feed ratio is from 4:1 to 1:1.

9. A process according to any one of claims 1 to 8 wherein the catalyst is selected from the group of Al₃, CrF₃, FeF₃, SbF₅, NiF₂, BF₃, SnF₄ and combination thereof.

10. The process according to any one of claims 1 to 9 wherein said process is a continuous process.

## Patentansprüche

1. Verfahren zur Herstellung von Vinylidenfluorid, dadurch **gekennzeichnet,** daß:
man ein Dampfphasen-Reaktionsgemisch, das Fluorwasserstoff und mindestens einen halogenierten Kohlenwasserstoff der Formel CHₙCXₘ enthält, worin n und m 2 oder 3 sind, und X jeweils unabhängig aus der aus Fluor, Chlor, Brom und Iod bestehenden Gruppe ausgewählt wird, herstellt,
man 1,1,1-Trifluorethan und/oder mindestens einen Teil des bei dem Verfahren als ein Reaktionsprodukt hergestellten 1,1,1-Trifluorethans dem Dampfphasen-Reaktionsgemisch als Verdünnungsmittel zusetzt,
man den Fluorwasserstoff und den halogenierten Kohlenwasserstoff in einer Reaktionszone in Gegenwart eines heterogenen Katalysators umsetzt, und
man Vinylidenfluorid als ein Reaktionsprodukt erhält.

2. Verfahren nach Anspruch 1, dadurch **gekenn****zeichnet,** daß der halogenierte Kohlenwasserstoff aus Vinylidenchlorid, 1,1,1-Trichlorethan und Gemischen daraus ausgewählt wird.

3. Verfahren nach Anspruch 1 oder 2, dadurch **gekennzeichnet**, daß die Reaktionstemperatur in der Reaktionszone von 400°C bis 650°C reicht.

4. Verfahren nach Anspruch 3, dadurch **gekennzeichnet**, daß die Reaktionstemperatur in der Reaktionszone von 550°C bis 625°C reicht.

5. Verfahren nach einem der Ansprüche 1 bis 4, dadurch **gekennzeichnet**, daß der halogenierte Kohlenwasserstoff und der Fluorwasserstoff für einen Zeitraum von 1 Sekunde bis 20 Sekunden in der Reaktionszone in Kontakt sind.

6. Verfahren nach Anspruch 5, dadurch **gekennzeichnet**, daß der halogenierte Kohlenwasserstoff und der Fluorwasserstoff für eine Zeit von 5 Sekunden bis 20 Sekunden in der Reaktionzone in Kontakt sind.

7. Verfahren nach einem der Ansprüche 1 bis 6, dadurch **gekennzeichnet** daß man das Dampfphasen-Reaktionsgemisch dadurch bildet, daß man Fluorwasserstoff und den halogenierten Kohlenwasserstoff in die Reaktionszone mit einem molaren Verhältnis der Einspeisung von 0:1 bis 10:1 einspeist.

8. Verfahren nach Anspruch 7, dadurch **gekennzeichnet**, daß das Molverhältnis der Einspeisung von 4:1 bis 1:1 reicht.

9. Verfahren nach einem der Ansprüche 1 bis 8, dadurch **gekennzeichnet**, daß man den Katalysator aus der Gruppe von AlF₃, CrF₃, FeF₃, SbF₅, NiF₂, BF₃, SnF₄ und Kombinationen daraus auswählt.

10. Verfahren nach einem der Ansprüche 1 bis 9, dadurch **gekennzeichnet** daß das Verfahren ein kontinuierliches Verfahren ist.

## Revendications

1. Procédé de production de fluorure de vinylidène, comprenant :
la formation d'un mélange réactionnel en phase vapeur comprenant du fluorure d'hydrogène et au moins un hydrocarbure halogéné de formule CHₙCXₘ dans laquelle n et m sont égaux à 2 ou 3 et chaque groupe X est choisi indépendamment entre le fluor, le chlore, le brome et l'iode ;
l'addition de 1,1,1-trifluoréthane et/ou d'au moins une portion du 1,1,1-trifluoréthane formé comme produit de réaction dans ledit procédé au mélange réactionnel en phase vapeur, comme diluant ;
la réaction du fluorure d'hydrogène et de l'hydrocarbure halogéné dans une zone réactionnelle en présence d'un catalyseur hétérogène ; et
l'obtention de fluorure de vinylidène comme produit de réaction.

2. Procédé suivant la revendication 1, dans lequel l'hydrocarbure halogéné est choisi dans le groupe comprenant le chlorure de vinylidène, le l,l,l-trichloréthane et leurs mélanges.

3. Procédé suivant la revendication 1 ou 2, dans lequel la température de réaction dans la zone réactionnelle est comprise dans l'intervalle de 400°C à 650°C.

4. Procédé suivant la revendication 3, dans lequel la température de réaction dans la zone réactionnelle est comprise dans l'intervalle de 550°C à 625°C.

5. Procédé suivant l'une quelconque des revendications 1 à 4, dans lequel l'hydrocarbure halogéné et le fluorure d'hydrogène sont en contact dans la zone réactionnelle pendant un temps de 1 seconde à 20 secondes.

6. Procédé suivant la revendication 5, dans lequel l'hydrocarbure halogéné et le fluorure d'hydrogène sont en contact dans la zone réactionnelle pendant un temps de 5 secondes à 20 secondes.

7. Procédé suivant l'une quelconque des revendications 1 à 6, dans lequel le mélange réactionnel en phase vapeur est formé par introduction de fluorure d'hydrogène et de l'hydrocarbure halogéné dans la zone réactionnelle, en un rapport molaire d'alimentation de 0:1 à 10:1.

8. Procédé suivant la revendication 7, dans lequel le rapport molaire d'alimentation est compris dans l'intervalle de 4:1 à 1:1.

9. Procédé suivant l'une quelconque des revendications 1 à 8, dans lequel le catalyseur est choisi dans le groupe comprenant AlF₃, CrF₃, FeF₃, SbF₆, NiF₂, BF₃, SnF₄ et leurs mélanges.

10. Procédé suivant l'une quelconque des revendications 1 à 9, qui est un procédé continu.
